# EUROPEAN PATENT SPECIFICATION

(11) **EP 2 022 860 B1**
(45) Date of publication and mention of the grant of the patent: **21.09.2011**
(21) Application number: 07014895.2
(22) Date of filing: 30.07.2007
(51) Int. Cl.: C12Q 1/68

(54) **An oligonucleotide probe and a process for detecting or discriminating microorganisms in a sample**
Oligonukleotidsonde und Verfahren zum Nachweis oder zur Unterscheidung von Mikroorganismen in einer Probe
Sonde oligonucléotide et procédé pour détecter ou discriminer des microorganismes dans un échantillon

(43) Date of publication of application: 11.02.2009
(73) Proprietor: Universität Ulm, 89081 Ulm (DE)
(72) Inventor: Poppert, Sven, Dr. med., 89075 Ulm (DE); Wellinghausen, Nele, 89075 Ulm (DE); Essig, Andreas, 89079 Ulm (DE); Riecker, Melanie, 89075 Ulm (DE)
(74) Representative: Pfenning, Meinig & Partner GbR

(56) References cited:
- WO-A1-2004/053156
- DATABASE Geneseq [Online] 21 August 2001 (2001-08-21), "Enterococcus sp detecting probe Entfae." XP002464047 retrieved from EBI accession no. GSN:AAH20853 Database accession no. AAH20853 & WO 01/36673 A (CREATOGEN AG [DE]; APFEL HEIKO [DE]; HEESEMANN JUERGEN [DE]; TREBESIUS) 25 May 2001 (2001-05-25)
- DATABASE Geneseq [Online] 29 July 2004 (2004-07-29), "Nucleobase portion of Enterococcus faecium peptide nucleic acid probe." XP002464048 retrieved from EBI accession no. GSN:ADO47139 Database accession no. ADO47139
- DATABASE EMBL [Online] 22 March 2003 (2003-03-22), "Sequence 10 from Patent WO02102824." XP002464049 retrieved from EBI accession no. EMBL:AX659408 Database accession no. AX659408 & WO 02/102824 A (VERMICON AG [DE]; BEIMFOHR CLAUDIA [DE]; SNAIDR JIRI [DE]) 27 December 2002 (2002-12-27)
- DATABASE Geneseq [Online] 6 April 2006 (2006-04-06), "Pathogenic bacteria detection 23S rRNA probe SEQ ID NO:4." XP002464050 retrieved from EBI accession no. GSN:AEF87565 Database accession no. AEF87565
- DATABASE Geneseq [Online] 6 November 2003 (2003-11-06), "Enterococcus faecium 23S rRNA probe DB6." XP002464051 retrieved from EBI accession no. GSN:ACF05441 Database accession no. ACF05441
- DATABASE Geneseq [Online] 26 July 2007 (2007-07-26), "Microorganism identification oligonucleotide SEQ ID NO 397." XP002464052 retrieved from EBI accession no. GSN:AGB08466 Database accession no. AGB08466 & WO 2007/056680 A (FORSYTH DENTAL IN RY FOR CHILD [US]; DEWHIRST FLOYD E [US]; PASTER BRU) 18 May 2007 (2007-05-18)
- DATABASE Geneseq [Online] 15 July 2004 (2004-07-15), "Oligo 7-3 used to diagnose Enterococcus faecium pathogen." XP002464053 retrieved from EBI accession no. GSN:ADO41373 Database accession no. ADO41373 & US 2004/010129 A1 (HSU PO-HSIANG [TW] ET AL) 15 January 2004 (2004-01-15)
- DATABASE Geneseq [Online] 29 July 2004 (2004-07-29), "Nucleobase portion of Enterococcus faecium peptide nucleic acid probe." XP002464054 retrieved from EBI accession no. GSN:ADO47138 Database accession no. ADO47138 & WO 2004/039831 A (ADVANDX INC [US]; STENDER HENRIK [US]) 13 May 2004 (2004-05-13)
- DATABASE Geneseq [Online] 26 July 2007 (2007-07-26), "Prokaryota operational taxonomic unit (OTU) specific probe, 87P." XP002464055 retrieved from EBI accession no. GSN:AGB06784 Database accession no. AGB06784 & US 7 214 492 B1 (RUBLEE PARKE A [US] ET AL) 8 May 2007 (2007-05-08)
- DATABASE Geneseq [Online] 27 July 2006 (2006-07-27), "Enterococcus avium 16S rRNA-specific probe 1, SEQ ID NO:36." XP002464056 retrieved from EBI accession no. GSN:AEH49468 Database accession no. AEH49468
- DATABASE EMBL [Online] 9 November 2005 (2005-11-09), "A probe for identifying a microorganism and a method for identifying a microorganism using the same." XP002464057 retrieved from EBI accession no. EMBL:BD409060 Database accession no. BD409060
- DATABASE Geneseq [Online] 28 June 2007 (2007-06-28), "E. faecium 23S RNA gene PCR primer/probe SEQ ID NO:20." XP002464058 retrieved from EBI accession no. GSN:AFC29681 Database accession no. AFC29681 & WO 2007/023461 A (PHILIPS INTELLECTUAL PROPERTY [DE]; KONINKL PHILIPS ELECTRONICS NV [NL) 1 March 2007 (2007-03-01)
- DATABASE Geneseq [Online] 29 July 2004 (2004-07-29), "Enterococcus faecium peptide nucleic acid blocker probe." XP002464059 retrieved from EBI accession no. GSN:ADO47140 Database accession no. ADO47140
- WERNER ET AL.: "Detection of Mutations conferring resistance to Linezolid in Enterococcus spp. by Fluorescence In Situ Hybridization", JOURNAL OF CLINICAL MICROBIOLOGY, vol. 45, no. 10, 2 July 2007 (2007-07-02), pages 3421-3423,

## Description

The present invention refers to oligonucleotide probes hybridizing with ribosomal RNA of microorganisms and a diagnostic kit comprising said oligonucleotide probes. Furthermore, the invention refers to a process for detecting and/or discriminating at least one microorganism in a sample. These oligonucleotide probes, in particular, can be used for the detection of bacteria of the genus *enterococcus.*

*Enterococci* are frequent causes of nosocomial infections like urinary tract infections and sepsis. Rapid identification of *enterococci* to species level is important for treatment decisions because different species show different resistance patterns. While most strains of *E*. *faecalis* are susceptible to penicillins, most strains of *E*. *faecium* require use of a glycopeptide antibiotic in first-line therapy. In turn, members of the the *VanC* type resistance group, such as *E. gallinarum* and *E. casseliflavus,* are intrinsically resistant against *vancomycin* but are mainly susceptible to penicillins. Species identification of *enterococci* by phenotypical methods is time consuming, however, and misidentification of *E. faecium* as *E. gallinarum* or *E. casseliflavus* and vice versa is a frequent problem.

In general, enterococci are identified by testing their biochemical metabolic characteristics, e.g. the ability to degrade specific sugars. Commercially available systems are used therefore, e.g. API. A disadvantage of these systems is their long measurement period as well as the low liability of identifying species, e.g. *E. gallinarum* or *E. faecium.* The low reproducibility is problematic due to the different resistance patterns of the different microbial strains. As an example, *E. faecium* mostly is resistant to ampicillin but sensitive to vancomycin while *VanC enterococci* as *E. gallinarum* are resistant to vancomycin but sensitive to ampicillin.

The Fluorescence in situ hybridization (FISH) is neither a time-consuming nor a complex biomolecular method. FISH was originally established in the tumor research. 15 years ago, FISH was modified for detecting bacteria. In general, for the detection of bacteria with FISH, oligonucleotide probes having a length of 15 to 30 base pairs are used. The probes will bind to specific complementary fragments of the ribosomal RNA and in this way label the targeting organism for fluorescence detection. FISH is already used for the detection of microorganisms in complex biological samples such as sludge. 6 years ago, the first steps for the detection of pathogenic bacteria in the medical microbiology were initiated, e.g. the detection of pathogens in blood cultures and cerebrospinal fluid (Kempf, V. A., K. Trebesius, and I. B. Autenrieth, 2000, "Fluorescent in situ hybridization allows rapid identification of microorganisms in blood cultures", J. Clin. Microbiol. 38:830-838; Moter et al., "Fluorescence in situ hybridization (FISH) for direct visualization of microorganisms", 2000, 41(2), p. 85-112).

The advantages of FISH are the fast measurement (< 2 h) as well as the specific and quantitative determination of pathogens in a sample. As FISH does not rely on any amplification step, it can be processed in a laboratory without any safeguards with respect to contamination. By combining probes having different coloured labels, the screening of up to 16 bacteria in a sample can be conducted on a single multiple field slide with 8 fields. Furthermore, FISH is very efficient with respect to the costs.

FISH using fluorescently labelled oligonucleotide probes targeting the ribosomal RNA is a rapid and easy-to-perform method that has already been used for detection of *E. faecalis* and *E. faecium* in blood cultures (Kempf, V. A., K. Trebesius, and I. B. Autenrieth, 2000, "Fluorescent in situ hybridization allows rapid identification of microorganisms in blood cultures", J. Clin. Microbiol. 38:830-838; Peters, R. P., P. H. Savelkoul, A. M. Simoons-Smit, S. A. Danner, C. M. Vandenbroucke-Grauls, and M. A. van Agtmael, 2006, "Faster identification of pathogens in positive blood cultures by fluorescence in situ hybridization in routine practice", J. Clin. Microbiol. 44:119-123). The published Enterococcus-probes have not been thoroughly evaluated, however, and are partly not specific according to a current GenBank database analysis. Others are implemented under unusual conditions such as formamide concentrations of either 10 % or 25 % or a hybridization temperature of 50 °C and may therefore not be combined with other DNA-FISH probes under standard hybridization conditions.

The publication by Werner et al., Journal of Clinical Microbiology (10/2007), pages 3421 - 3423, provides detection and discrimination of Enterococcus species using FISH together with LNA probes. In addition linezolid resistance is detected with probes that are identical to the ones used in the underlying application. In contrast to the underlying application however this publication is silent on the FISH-probes used for the individual Enterococci-strains and furthermore the spectrum of strains detected is much smaller than in the underlying application.

It was an object of the present invention to provide a process for detecting microorganisms, in particularly clinically relevant *enterococci* having a high sensitivity and selectivity to these microorganisms.

This object was solved by an oligonucleotide probe having the features of claim 1, a diagnostic kit with the features of claim 10 and a process having the features of claim 14. The further dependent claims show preferred embodiments.

According to the present invention, an oligonucleotide probe is provided, specific for hybridizing with ribosomal RNS of microorganisms, said probe comprising at least one oligonucleotide having a sequence at least 80 % homologous to at least one of SEQ ID NO. 1 to 16 including sequences extended or deleted by one or more nucleotides:
SEQ ID NO. 1: GAA AGC GCC TTT CAC TCT TAT GC,
SEQ ID NO. 2: TAG GTG TTG TTA GCA TTT CG,
SEQ ID NO. 3: GAC TCC TTC AGA CTT ACT GCT TGG,
SEQ ID NO. 4: TTC ACA CAA TCG TAA CAT CCT A,
SEQ ID NO. 5: ATT CAC AAC TGT GTA ACA TCC TAT,
SEQ ID NO. 6: CAA AAG CGC CTT TCA AAC CGA,
SEQ ID NO. 7: TTG TCC GTA CAG GTA GAA GCA T,
SEQ ID NO. 8: AGC TTG TCC GTA CAG GTA GAG AA,
SEQ ID NO. 9: TCA CGC AAA CGT AAC ATC C,
SEQ ID NO. 10: TCA GCG GCA CCG TAA AG,
SEQ ID NO. 11: TTT TCC CGG TGG AGC AA,
SEQ ID NO. 12: CAG TTA CTC TCA CCC TTG TTC TT,
SEQ ID NO. 13: CAG TTC TCT GCG TCT ACC TC,
SEQ ID NO. 14: CAC CGC GGG TCC ATC CAT CA,
SEQ ID NO. 15: CCC TCT GAT GGG TAG GTT, and
SEQ ID NO. 16: CAA CTT TCT TCC ATG CGG AAA AT.

It is preferred that the microorganisms are selected from the genus *Enterococcus,* in particular from the genera *Enterococcus faecalis, Enterococcus faecium, Enterococcus gallinarium, Enterococcus avium, Enterococcus casseliflavus*, *Enterococcus hirae*, *Enterococcus durans, Enterococcus mundtii, Enterococcus asini, Enterococcus cecorum ,Enterococcus columbae, Enterococcus dispar*, *Enterococcus gilvus*, *Enterococcus haemoperoxidans, Enterococcus malodoratus, Enterococcus moraviens, Enterococcus pallens, Enterococcus porcinus*, *Enterococcus pseudoavium*, *Enterococcus raffinosus, Enterococcus ratti, Enterococcus saccharolyticus, Enterococcus seriolicida, Enterococcus solitarius*, *Enterococcus sulfureus* and *Enterococcus villorum.*

The probe preferably further comprises at least one detectable marker. This detectable marker is preferably covalently bound to the oligonucleotide. The detectable marker preferably is a fluorescence marker, a chemoluminescence marker, a radioactive marker, an enzymatically active group, a hapten and/or a nucleic acid detectable by hybridization.

In a preferred embodiment, the oligonucleotide has a sequence at least 90 %, preferably 95 % and more preferably 100 % homologous to at least one of SEQ ID NO. 1 to 16.

It is preferred that the oligonucleotide further comprises a sequence at least 80 % homologous to SEQ ID NO. 24 including sequences extended or deleted by one or more nucleotides:
SEQ ID NO. 24: CCA GCT AGC GTG C,
and at least the nucleotide at the underlined position in SEQ ID NO. 24 is a locked nucleic acid (LNA) such that the oligonucleotide probe specifically detects a point mutation of the microorganism under conditions which permit the probes to hybridize specifically, and determining the appearance of in situ hybridization between said probes and the microorganism in said sample as an indication of linezolid resistance.

In a further preferred embodiment, the oligonucleotide further comprises a sequence at least 80 % homologous to SEQ ID NO. 25 including sequences extended or deleted by one or more nucleotides:
SEQ ID NO. 25: CCA GCT CGC GTG C,
and at least the nucleotide at the underlined position in SEQ ID NO. 25 is a locked nucleic acid (LNA) such that the oligonucleotide probe specifically detects a Linezolid wildtype.

The invention further comprises a diagnostic kit comprising at least one oligonucleotide probe of any of the oligonucleotide probes mentioned in claims 1 to 9.

It is preferred that the diagnostic kit further comprises a competitive probe comprising an oligonucleotide having a sequence at least 80 % homologous to at least one of SEQ ID NO. 17 to 23 including sequences extended or deleted by one or more nucleotides:
SEQ ID NO. 17: CGA AAG CGC CTT TCA AAT CAA being competitive to SEQ ID NO. 6,
SEQ ID NO. 18: TTG TCC GTA CAG ATA GAA GCA T being competitive to SEQ ID NO. 7,
SEQ ID NO. 19: TCA CGC AGA CGT AAC ATC C being competitive to SEQ ID NO. 9,
SEQ ID NO. 20: TTT TTC CGG TGG AGC AA being competitive to SEQ ID NO. 11,
SEQ ID NO. 21: CGG CGC GGG TCC ATC CAG AA being competitive to SEQ ID NO. 14,
SEQ ID NO. 22: ACG CCG CGG GTC CAT CCA TWA being competitive to SEQ ID NO. 14, and
SEQ ID NO. 23: GTT TCA GTT CCC CRC GTC TAC CT being competitive to SEQ ID NO. 13.

These oligonucleotide probes are useful for a competitive assay, in particular a FISH assay. The competitive probes are added without a marker together with the corresponding probes mentioned above, which comprise at least one detectable marker. The competitive probes are bound to the complementary ribosomal sequences of non-target organisms and prevent the binding of marked probes to these organisms.

The diagnostic kit preferably comprises a further oligonucleotide probe, said probe comprising at least one oligonucleotide having a sequence at least 80 % homologous to SEQ ID NO. 24 including sequences extended or deleted by one or more nucleotides:
SEQ ID NO. 24: CCA GCT AGC GTG C,
and at least the nucleotide at the underlined position in SEQ ID NO. 24 is a locked nucleic acid (LNA) such that the further oligonucleotide probe specifically detects a point mutation of the microorganism under conditions which permit the probes to hybridize specifically and determins the appearance of in situ hybridization between said probes and the microorganism in said sample as an indication of linezolid resistance.

Furthermore, it is preferred that the diagnostic kit comprises a further oligonucleotide probe, said probe comprising at least one oligonucleotide having a sequence at least 80 % homologous to SEQ ID NO. 25 including sequences extended or deleted by one or more nucleotides:
SEQ ID NO. 25: CCA GCT CGC GTG C,
and at least the nucleotide at the underlined position in SEQ ID NO. 25 is a locked nucleic acid (LNA) for detection of the linezolid wildtype.

Furthermore, the present invention comprises a process for detecting and/or discriminating at least one microorganism in a sample. This process comprises as a first step the incubation of a sample with at least one oligonucleotide probe comprising at least one oligonucleotide having a sequence at least 80 % homologous to at least one of SEQ ID NO. 1 to 16 including sequences extended or deleted by one or more nucleotides.

A further step of this process comprises the determination of the presence of microorganisms hybridized with the probe.

It is preferred that the probe further comprises at least one detectable marker which preferably is covalently bound to the oligonucleotide probe. The type of detectable marker is not limited such that any known detectable marker can be used. Preferred detectable markers are selected from the group consisting of fluorescence marker, chemoluminescence marker, radioactive marker, enzymatically active group, hapten and nucleic acids detectable by hybridization.

The inventive process in particular is conducted as a FISH assay. For such an assay, fluorescein or cyanin dyes are preferred as a marker.

In a preferred embodiment, the process comprises a quantification of at least microorganism. A microorganism preferably belongs to the genus *enterococcus,* in particular. More preferably, the at least one microorganism belongs to the genera of Van-C group *enterococci,* in particular *Enterococcus gallinarium, Enterococcus casseliflavus* and/or *Enterococcus flavescens.*

It is further possible that additionally to the said oligonucleotide probes, at least one competitive probe which is unmarked is used. The competitive probe preferably comprises an oligonucleotide having a sequence at least 80 % homologous to at least one of SEQ ID NO. 17 to 23 including sequences extended or deleted by one or more nucleotides:
SEQ ID NO. 17: CGA AAG CGC CTT TCA AAT CAA being competitive to SEQ ID NO. 6,
SEQ ID NO. 18: TTG TCC GTA CAG ATA GAA GCA T being competitive to SEQ ID NO. 7,
SEQ ID NO. 19: TCA CGC AGA CGT AAC ATC C being competitive to SEQ ID NO. 9,
SEQ ID NO. 20: TTT TTC CGG TGG AGC AA being competitive to SEQ ID NO. 11,
SEQ ID NO. 21: CGG CGC GGG TCC ATC CAG AA being competitive to SEQ ID NO. 14,
SEQ ID NO. 22: ACG CCG CGG GTC CAT CCA TWA being competitive to SEQ ID NO. 14, and
SEQ ID NO. 23: GTT TCA GTT CCC CRC GTC TAC CT being competitive to SEQ ID NO. 13.

These oligonucleotide probes are useful for a competitive assay, in particular a FISH assay. The competitive probes are added without a marker together with the corresponding probes mentioned above, which comprise at least one detectable marker. The competitive probes are bound to the complementary ribosomal sequences of non-target-organisms and prevent the binding of marked probes to these organisms.

Furthermore, it is preferred that said oligonucleotide probe also specifically detects a point mutation of microorganisms under conditions which permit the probes to hybridize specifically, and determining the appearance of in situ hybridization between said probes and the microorganism in said sample as an indication of linezolid resistance.

The present process thereby enables the simultaneous detection of linezolid resistance of microorganisms by using one of the above-mentioned oligonucleotide probes with SEQ ID NO. 1 to 16 which further comprise at least one oligonucleotide having a sequence at least 80 % homologous to SEQ ID NO. 24 including sequences extended or deleted by one or more nucleotides:
SEQ ID NO. 24: CCA GCT AGC GTG C
and at least the nucleotide at the underlined position in the general formula is a locked nucleic acid (LNA). It is further preferred that the oligonucleotide probes further comprise at least one oligonucleotide having a sequence at least 80 % homologous to SEQ ID NO. 25 including sequences extended or deleted by one or more nucleotides:
SEQ ID NO. 25: CCA GCT CGC GTG C,
and at least the nucleotide of the underlined position in the general formula is a locked nucleic acid (LNA) which can be used for the detection of the linezolid wildtype.

In a preferred embodiment of the inventive process, this process can be combined with a simultaneous detection of linezolid resistance of the microorganism by contacting the sample with a further oligonucleotide probe, which specifically detects a point mutation under conditions which permit the probes to hybridize specifically, and determining the appearance of in situ hybridization between said probes and the microorganism in said sample as an indication of linezolid resistance.

It is preferred that the further oligonucleotide probe comprises at least one oligonucleotide having a sequence at least 80 % homologous to SEQ ID NO. 24 including sequences extended or deleted by one or more nucleotides:
SEQ ID NO. 24: CCA GCT AGC GTG C.

This oligonucleotide probe is used for the detection of linezolid resistance of microorganisms in the sample.

Furthermore, it is preferred to add oligonucleotide probes with at least one oligonucleotide having a sequence at least 80 % homologous to SEQ ID NO. 25 including sequences extended or deleted by one or more nucleotides:
SEQ ID NO. 25: CCA GCT CGC GTG C.

This oligonucleotide probe is used for the detection of linezolid wildtype.

A summary of said oligonucleotide probes is shown in table 1.

**Table 1**

| ***Probe name*** | ***Target organism*** | ***SEQ No.*** | ***Sequence*** |
|---|---|---|---|
| ENF 16S 191 | Enterococcus faecalis | 1 | GAA AGC GCC TTT CAC TCT TAT GC |
| ENF 23S 342 | Enterococcus faecalis | 2 | TAG GTG TTG TTA GCA TTT CG |
| ENU 23S 1470 | Enterococcus faecium | 3 | GAC TCC TTC AGA CTT ACT GCT TGG |
| NEU 140 | Enterococcus faecium | 4 | TTC ACA CAA TCG TAA CAT CCT A |
| EGA 16S 141 | Enterococcus gallinarum | 5 | ATT CAC AAC TGT GTA ACA TCC TAT |
| E. av 16S 193 | Enterococcus avium | 6 | CAA AAG CGC CTT TCA AAC CGA |
| E. cassel 23S 1495 | Enterococcus casseliflavus | 7 | TTG TCC GTA CAG GTA GAA GCA T |
| E. cassel 23S 1499 | Enterococcus casseliflavus | 8 | AGC TTG TCC GTA CAG GTA GAG AA |
| E. hirae dur 23S 141 | Enterococcus hirae durans | 9 | TCA CGC AAA CGT AAC ATC C |
| E. mundtii 16S 201 (Emu 201) | Enterococcus mundtii | 10 | TCA GCG GCA CCG TAA AG |
| E. mundtii 16S 82 (Emu 82) | Enterococcus mundtii | 11 | TTT TCC CGG TGG AGC AA |
| E. mundtii 16S (Emu 16S) | Enterococcus mundtii | 12 | CAG TTA CTC TCA CCC TTG TTC TT |
| ENC 176 23S kurz | Enterococcus Genus | 13 | CAG TTC TCT GCG TCT ACC TC |
| ENC 16S 221 | Enterococcus Genus | 14 | CAC CGC GGG TCC ATC CAT CA |
| Entalle 23S | Enterococcus Genus | 15 | CCC TCT GAT GGG TAG GTT |
| Egac 16S 183 | Enterococcus gallinarum, casselifavus, flavescens | 16 | CAA CTT TCT TCC ATG CGG AAA AT |
| E. av KOMP 16S 193 | Enterococcus avium competitor | 17 | CGA AAG CGC CTT TCA AAT CAA |
| E. cassel 23S 1495 KOMP | Enterococcus casseliflavus competitor | 18 | TTG TCC GTA CAG ATA GAA GCA T |
| E. hirae dur 23S 141-KOMP | Enterococcus hirae durans competitor | 19 | TCA CGC AGA CGT AAC ATC C |
| Komp E. mundtii 16S 82 (Emu K 82) | Enterococcus mundtii competitor | 20 | TTT TTC CGG TGG AGC AA |
| ENC 16S 221 Komp | Enterococcus Genus competitor 1 | 21 | CGG CGC GGG TCC ATC CAG AA |
| ENC 16S 222 Komp Carno | Enterococcus Genus competitor 2 | 22 | ACG CCG CGG GTC CAT CCA TWA |
| ENC 176 23S Komp 1+2 | Enterococcus Genus competitor | 23 | GTT TCA GTT CCC CRC GTC TAC CT |
| ENC Lin resistent | ENC Linezolid | 24 | CCA GCT AGC GTG C |
| ENC Lin sensibel | ENC Linezolid | 25 | CCA GCT CGC GTG C |

The following examples illustrate preferred embodiments of the invention, not limiting the present invention to these specific embodiments.

### Example 1

FISH probes were designed including species-specific probes for *E. faecium*, *E*. *faecalis*, and *E*. *gallinarum*, a group-specific probe for the *VanC*-type resistance group, and two genus-specific probes for all enterococci. Probes were designed using the ARB software and directly 5'-labeled with 5(6)-carboxy-fluorescein-N-hydroxysuccinimide ester (FLUOS) or the sulphoindocyanine dye Cy3 (Thermo, Ulm, Germany). All probes were used at a formamide concentration of 30 % as described previously (Peters, R. P., M. A. van Agtmael, A. M. Simoons-Smit, S. A. Danner, C. M. Vandenbroucke-Grauls, and P. H. Savelkoul, 2006, "Rapid identification of pathogens in blood cultures with a modified fluorescence in situ hybridization assay", J. Clin. Microbiol. 44:4186-4188). This allows simultaneous implementation of all probes on multiple field slides.

In the first step, the probes were evaluated on 14 *Enterococcus* spp. and 19 non-enterococcal reference strains (ATCC no. in parentheses): *E. avium* (14025), *E. casseliflavus* (25788, 12755), *E. durans* (20633), *E. faecalis* (29212, *51299,* 19433), *E. faecium* (19434), *E*. *flavescens* (7370), *E. gallinarum* (700425, 35038), *E. hirae* (8043), *E. mundtii* (43186), *E. raffinosus* (49427), *Streptococcus agalactiae* (13813), *S*. *anginosus (12395), S. bovis* (33317), *S.* constellatus (27823), *S. mitis* (49456), *S. mutans* (25175), S. *oralis (35037), S. parasanguis* (15912), *S. pneumoniae* (6303, 49619, 20566, 11865), *S. pyogenes* (12344), *S*. *salivarius* (7073), *S. sanguis (10556), Staphylococcus aureus* (29213, 43300), *Staphlyococcus epidermidis* (12228), and *Staphylococcus saprophyticus* (15305). All probes correctly detected the respective target organisms. Apart from the probes ENC 176 and ENU 1470 that cross-reacted with *Staphylococcus saprophyticus* and one isolate of *Enterococcus durans,* respectively, all probes had an analytical specificity of 100 %. Because of the observed cross-reaction, five well-characterized isolates of *E. durans* were additionally included and did not show any cross-reaction. The probes were further evaluated on 68 clinical isolates, including 49 enterococcal strains and one *Abiotrophia adiacens* from blood cultures and 18 *vancomycin*-resistant strains grown on VRE screening agar (BD, Heidelberg, Germany), including three *E. faecium vanA,* one *E. faecium vanB,* and 14 *E. gallinarum* strains. The same *E. faecium vanB* strain (according to pulsed field gel electrophoresis) was recovered from 30 patients during an outbreak in our hospital. All isolates were investigated by FISH. The clinical isolates were identified by growth on bile-esculin agar with esculin hydrolysis, API 20 Strep (Bio-Mérieux, Nuertingen, Germany), formation of methyl-α-D-glucopyranoside (according to Devriese, L. A., B. Pot, K. Kersters, S. Lauwers, and F. Haesebrouck, 1996, "Acidification of methyl-alpha-D-glucopyranoside: a useful test to differentiate Enterococcus casseliflavus and Enterococcus gallinarum from Enterococcus faecium species group and from Enterococcus faecalis, J. Clin. Microbiol. 34:2607-2608), and pigmentation. Sequencing of the complete 16S rRNA gene was performed as described previously in isolates with ambiguous phenotypic identification results (n = 5). All FISH-probes consistently reacted in accordance with the identification results shown in table 2.

It was further assessed whether FISH may also be used for rapid identification of *enterococci* directly from positive blood cultures. Fifty-eight positive blood cultures microscopically showing gram-positive *diplococci* or *cocci* in chains suggestive of *enterococci* were included. One additional sample was investigated but was not included in the data analysis because the FISH slide contained no bacteria, most probably because of a mistake during sample preparation. For sample preparation, the blood culture medium was centrifuged at 130 x g for 10 min in order to remove erythrocytes. The supernatant was centrifuged again at 1800 x g for 5 min, and the bacterial pellet was suspended in 0.9 % saline. 10 µl of the suspension was applied to each field of an 8-well glass slide, air dried, and fixed in methanol for 10 min. Five of the 58 samples contained mixtures of different *enterococci* or *enterococci* and other bacteria. Including mixtures and pure cultures, 32 *Enterococcus* spp. and 28 *Streptococcus* spp. and related species were cultured. All *enterococci* were correctly identified to the species level by FISH, including the cases in mixed cultures (Table 1). All 28 non-*enterococcal* isolates gave correct negative results with the probes (*Streptococcus mitis* (n=6), S. *pneumoniae* (n=5), *S. oralis* (n=3), *S*. *pyogenes* (n=3), S. *sanguis* (n=2), *S*. anginosus (n=2), *S*. *salivarius* (n=1), *Gemella* spp. (n=3), *Lactococcus lactis* (n=2), and *Abiotrophia adiacens* (n=1)).

Altogether, the newly designed FISH-probe set proved highly sensitive and specific for rapid identification of clinically relevant enterococcal species in the clinical microbiology laboratory. Because the assay contains multiple probes in a hierarchical manner, rare cases of malfunctioning of single probes will become apparent through contradictory binding patterns. A considerable advantage of the FISH-assay is the possibility to discriminate reliably between *E. faecium* and *enterococci* of the *VanC* complex, which are often confused by commercial phenotypical methods.

Identification of these strains is of major importance because of their typical resistance patterns. In addition, FISH allows rapid differentiation of the *E. faecium* outbreak strain from VanC-harboring *E. gallinarum* isolates also growing on the VRE screening plates and showing ambiguous API results. The black pigmentation of the colonies caused by esculin hydrolysis did not interfere with the FISH-procedure. In addition to identification of enterococci from pure cultures, FISH proved highly useful for direct application on positive blood cultures, including even blood cultures with polymicrobial growth. The FISH-assay may, thus, significantly shorten the time to result in the clinical microbiology laboratory and to initiation of pathogen-adapted antimicrobial therapy, especially in septic patients.

**Table 2: Identification of clinical isolates by FISH**

| **Isolated species** | **Identification of clinical isolates (%)** | **Direct identification of enterococci in micro-scopically positive blood cultures (%)** |
|---|---|---|
| *E. faecium* | 23/23 (100) | 18/18 (100) |
| *E. faecalis* | 26/26 (100) | 12/12 (100) |
| *E. avium* | 3/3 (100)^{a} | -- |
| *E. gallinarum* | 15/15 (100) | 2/2 (100) |
| *Streptococci* | -- | 27/27^{b} |
| *Abiotrophia adiacens* | 1/1^{b} | 1/1^{b} |
| Total isolates | 68/68 (100) | 60/60 (100) |

| | | |
|---|---|---|
| ^{a}Correct identification as *Enterococcus spp.* other than *E. faecalis*, *E. faecium, E. gallinarum, E. casseliflavus* and *E. flavescens* ^{b}Correct identification as non-*Enterococcus* | | |

### Example 2

The reliability of FISH in detecting linezolid resistance in enterococci was evaluated in a blinded manner using 23 "pre-characterized" strains with known numbers of mutated alleles and known minimal inhibitory concentration (MIC) for linezolid (Table 3).

The applicability of the assay in a routine setting was subsequently determined using 83 clinical isolates (Table 4) .

Resistance to linezolid was defined by a MIC ≥ 8 mg/L according to DIN 58940, EUCAST 2006-06-20 V1.3, and CLSI Norm M100-S15. For the set of pre-characterized test strains, the linezolid MIC was determined by microbroth dilution using Iso-Sensitest broth as nutrient medium. For the clinical isolates, MIC of linezolid was determined by broth dilution using the Merlin MICRONAUT System (Merlin, Bornheim-Hesel, Germany). Single clinical isolates that were suspected to be linezolid-resistant were additionally tested by E-test (AB Biodisk, Solna, Sweden) and Iso-Sensitest. In all resistant isolates, the number of mutated 23S rDNA alleles was determined by a previously described method using LabChip technology and BioAnalyzer 2100.

For FISH, isolates were cultured on sheep blood agar for 18-24 h and suspended in 0.9% saline (McFarland 0.5). 10 µl of the suspension was applied to a glass slide, air dried, and fixed for 10 min in methanol. DNA-FISH-probes containing so-called locked nucleic acids (LNA) at the site of point mutation (LZD wildtype: FITC-5'-CCCAGCTCGCGTGC-3'; LZD resistant: Cy3-5'-CCAGCTAGCGTGC-3'; Thermo Hybaid, Ulm, Germany) were used, because these allow sharper discrimination of single-nucleotide alterations as described recently. FISH was performed at 46°C using 30% formamide in the hybridization buffer and the corresponding salt concentrations in washing buffer as described elsewhere (Amann, R. I., W. Ludwig and K. H. Schleifer, 1995, "Phylogenetic identification and in situ detection of individual microbial cells without cultivation", Microbiol. Rev. 59:143-169; and Manz W. and Amann R., Ludwig W., Wagner M., Schleifer K.-H., "Phylogenetic oligodeoxynucleotide probes for the major subclasses of Proteobacteria: problems and solutions", Syst. Appl. Microbiol. 1, 593-600, 2004). Hybridization was performed using a microwave (Sharp R208, SeaPro) approach as recently published. The bacterial probe EUB338 targeting nearly all bacteria was used as a positive control in all experiments. All FISH experiments were conducted in duplicate.

In preliminary experiments using eight previously investigated strains the presence of a single mutated allele led to a strong FISH signal with the respective probes. The signal was only marginally stronger when multiple alleles were mutated. FISH thus allowed detection of a single mutated allele but not assessment of the number of mutated alleles.

Twenty-three pre-characterized *Enterococcus* strains, 10 linezolid-sensitive and 13 linezolid-resistant, were then investigated in a blinded manner by FISH (Table 3). In both independent experiments, FISH showed correct results in all resistant and all susceptible strains (Table 3).

Finally, applicability of the assay was assessed using 83 clinical isolates grown in blood cultures or on VRE screening agar plates (Table 4). Seventy-nine isolates were found to be susceptible by FISH and microtiter broth dilution using the Micronaut system. One *E. faecium* blood culture isolate was determined as resistant by broth dilution and E-Test with a MIC of 8 mg/L. FISH showed a positive result with the resistance probe as well as with the wildtype probe, indicating the presence of mutated and non-mutated alleles, which was confirmed by LabChip technology. The strain was vancomycin sensitive and was isolated from the blood culture of a patient who had received tazobac but not linezolid.

Three *E. faecium* isolates gave a positive result with the linezolid-resistance probe, which were repeatedly determined to be phenotypically susceptible by microtiter broth dilution using the Micronaut system as well as the Iso-Sensitest system and by E-Test (Table 2, bold isolates). All three isolates showed a single mutated 23S rDNA allele by LabChip technology. One strain was vancomycin-resistant and was isolated from stool of a known carrier of a *vanA* type VRE. The patient had received linezolid for 10 days 5 months before isolation of the strain. Two vancomycin-susceptible strains were isolated from blood cultures from two patients treated at different intensive care units at different times. Both patients had received various antimicrobials such as imipenem, vancomycin, and piperacillin but not linezolid.

Considering primary phenotypic resistance testing, linezolid would have been a treatment option for the three patients. In view of previous reports on the exchange of mutated alleles via recombination in vitro under selective pressure, such strains might, however, be prone to rapid development of resistance. In order to test this hypothesis we performed in vitro mutagenesis experiments with the three strains in comparison to two linezolid-susceptible isolates possessing only wildtype 23S alleles. Exponentially grown cells were streaked onto agar plates supplemented with linezolid (2, 4, and 6 mg/L). Two of the three test strains, but none of the susceptible reference strains, showed single colonies that grew on plates with linezolid after 48 h incubation at 37°C. All four investigated representative colonies from plates with 4 and 6 mg/L linezolid showed linezolid MICs of 8 mg/L, indicating spontaneous resistance development. LabChip technology revealed that the number of resistant 23S alleles increased from one in the original strain to two in the mutants. Such isolates should therefore preferably not be treated with linezolid. If they are treated with linezolid, close monitoring for development of resistance is warranted by assessment of clinical improvement and by repeated sampling and, if applicable, testing of further isolates by phenotypic methods.

It is a crucial observation that the presence of a single mutated 23S allele leads to phenotypically detectable linezolid resistance in some (Table 1) but not all isolates (Table 2). It may be speculated that 23S alleles could be expressed heterogeneously, for instance, depending on the distance of the mutated 23S allele from the origin of replication, which might influence the amount of replicated mutated rRNA available for formation of mutated/linezolid-resistant ribosomes. As long as genome data for *E. faecium* is incomplete in this regard, this hypothesis remains speculative.

The occurrence of such strains even in patients who had never received linezolid stresses the need for molecular determination of linezolid resistance of enterococci, particularly in cases of elevated linezolid MIC values or unsatisfactory response to therapy with linezolid. FISH using LNA-containing probes proved to be a suitable and reliable method for this purpose. It showed 100% sensitivity for detection of phenotypic linezolid resistance and even allowed detection of a single mutated 23S allele in phenotypically linezolid-susceptible enterococci. As a rapid and easily performed method, FISH may also be implemented for initial determination of linezolid resistance or screening of sample collections. Additional advantages of FISH are the possibilities of simultaneously differentiating enterococci to species level using previously described FISH probes and of using the test directly on clinical samples, such as blood cultures (Wellinghausen et al., manuscript under revision). FISH thus has the potential to significantly improve and accelerate determination of linezolid resistance in *E. faecium.*

## Claims

1. A diagnostic kit for detecting and/or discriminating at least one microorganism from the genus *Enterococcus* in a sample by fluorescence in situ hybridization (FISH) comprising
a) at least one oligonucleotide probe specific for hybridizing with ribosomal RNA of microorganisms from the genus *Enterococcus,* said probe comprising at least one oligonucleotide having a sequence of SEQ ID NO. 1 to 16:
SEQ ID NO. 1: GAA AGC GCC TTT CAC TCT TAT GC,
SEQ ID NO. 2: TAG GTG TTG TTA GCA TTT CG,
SEQ ID NO. 3: GAC TCC TTC AGA CTT ACT GCT TGG,
SEQ ID NO. 4: TTC ACA CAA TCG TAA CAT CCT A,
SEQ ID NO. 5: ATT CAC AAC TGT GTA ACA TCC TAT,
SEQ ID NO. 6: CAA AAG CGC CTT TCA AAC CGA,
SEQ ID NO. 7: TTG TCC GTA CAG GTA GAA GCA T,
SEQ ID NO. 8: AGC TTG TCC GTA CAG GTA GAG AA,
SEQ ID NO. 9: TCA CGC AAA CGT AAC ATC C,
SEQ ID NO. 10: TCA GCG GCA CCG TAA AG,
SEQ ID NO. 11: TTT TCC CGG TGG AGC AA,
SEQ ID NO. 12: CAG TTA CTC TCA CCC TTG TTC TT,
SEQ ID NO. 13: CAG TTC TCT GCG TCT ACC TC,
SEQ ID NO. 14: CAC CGC GGG TCC ATC CAT CA,
SEQ ID NO. 15: CCC TCT GAT GGG TAG GTT, and
SEQ ID NO. 16: CAA CTT TCT TCC ATG CGG AAA AT,
b) at least one competitive probe comprising an oligonucleotide having a sequence of SEQ ID NO. 17 to 23
SEQ ID NO. 17: CGA AAG CGC CTT TCA AAT CAA being competitive to SEQ ID NO. 6,
SEQ ID NO. 18: TTG TCC GTA CAG ATA GAA GCA T being competitive to SEQ ID NO. 7,
SEQ ID NO. 19: TCA CGC AGA CGT AAC ATC C being competitive to SEQ ID NO. 9,
SEQ ID NO. 20: TTT TTC CGG TGG AGC AA being competitive to SEQ ID NO. 11,
SEQ ID NO. 21: CGG CGC GGG TCC ATC CAG AA being competitive to SEQ ID NO. 14,
SEQ ID NO. 22: ACG CCG CGG GTC CAT CCA TWA being competitive to SEQ ID NO. 14, and
SEQ ID NO. 23: GTT TCA GTT CCC CRC GTC TAC CT being competitive to SEQ ID NO. 13,
c) at least one further oligonucleotide probe or at least one of said oligonucleotide probes a) and b) with at least one oligonucleotide comprising a sequence of SEQ ID NO. 24:
SEQ ID NO. 24: CCA GCT AGC GTG C,
and at least the nucleotide at the underlined position in SEQ ID NO. 24 is a locked nucleic acid (LNA) such that the oligonucleotide probe specifically detects a point mutation of the microorganism under conditions which permit the probes to hybridize specifically, and determining the appearance of in situ hybridization between said probes and the microorganism in said sample as an indication of linezolid resistance, and
d) at least one further oligonucleotide probe or at least one of said oligonucleotide probes a) and b) with at least one oligonucleotide comprising a sequence of SEQ ID NO. 25:
SEQ ID NO. 25: CCA GCT CGC GTG C,
and at least the nucleotide at the underlined position in SEQ ID NO. 25 is a locked nucleic acid (LNA) such that the oligonucleotide probe specifically detects a Linezolid wildtype.

2. The diagnostic kit of claim 1,
wherein the microorganisms are selected from the genera *Enterococcus faecalis, Enterococcus faecium*, *Enterococcus gallinarium, Enterococcus avium, Enterococcus casseliflavus, Enterococcus flavescens, Enterococcus hirae*, *Enterococcus durans,* and *Enterococcus mundtii*.

3. The diagnostic kit of any of the preceding claims,
wherein said probe a) further comprises at least one detectable marker.

4. The diagnostic kit of claim 3,
wherein the detectable marker is covalently bound to said probe a).

5. The diagnostic kit of claim 3 or 4,
wherein the detectable marker is a fluorescence marker, a chemoluminescence marker, a radioactive marker, an enzymatically active group, a hapten and/or a nucleic acid detectable by hybridization.

6. The diagnostic kit of any of the preceding claims,
wherein the competitive probe is unmarked.

7. A process for detecting and/or discriminating at least one microorganism from the genus *Enterococcus* in a sample, comprising incubating the sample with the oligonucleotide probes of the diagnostic kit of one of the preceding claims and determining by fluorescence in situ hybridization (FISH) the presence of microorganisms hybridized with the probe, wherein the oligonucleotide of said probe a) being SEQ ID No. 1 and/or SEQ ID No. 2 detecting the microorganism *Enterococcus faecalis* and/or
the oligonucleotide of said probe a) being SEQ ID No. 3 and/or SEQ ID No. 4 detecting the microorganism *Enterococcus faecium* and/or
the oligonucleotide of said probe a) being SEQ ID No. 5 detecting the microorganism *Enterococcus gallinarium* and/or
the oligonucleotide of said probe a) being SEQ ID No. 6 detecting the microorganism *Enterococcus avium* and/or
the oligonucleotide of said probe a) being SEQ ID No. 7 and/or SEQ ID No. 8 detecting the microorganism *Enterococcus casseliflavus* and/or
the oligonucleotide of said probe a) being SEQ ID No. 9 detecting the microorganism *Enterococcus hirae* and/or *Enterococcus durans* and/or
the oligonucleotide of said probe a) being SEQ ID No. 10 to SEQ ID No. 12 detecting the microorganism *Enterococcus mundtii* and/or
the oligonucleotide of said probe a) being SEQ ID No. 13 detecting the genera *Enterococcus spp*. and *Carnobacterium spp*. and/or
the oligonucleotide of said probe a) being SEQ ID No. 14 detecting the genera *Enterococcus spp., Granulicatella spp*. and *Abiotrophia spp*. and/or the oligonucleotide of said probe a) being SEQ ID No. 15 detecting the genus *Enterococcus spp.* and/or
the oligonucleotide of said probe a) being SEQ ID No. 16 detecting the genera of Van-C group en*terococci*.

8. The process of claim 7,
further comprising quantifying the at least one microorganism.

9. The process of claim 7 or 8,
wherein the at least one microorganism belongs to the genera of Van-C group enterococci.

10. The process of one of the claims 7 to 9,
wherein the at least one microorganism belongs to the genera *Enterococcus faecalis*, *Enterococcus faecium, Enterococcus avium, Enterococcus hirae, Enterococcus durans,* and/or *Enterococcus mundtii.*

11. The process of any of the claims 7 to 10,
wherein the probe further comprises at least one detectable marker, preferably a fluorescence marker, a chemoluminescence marker, a radioactive marker, an enzymatically active group, a hapten and/or a nucleic acid detectable by hybridization.

12. The process of claim 11,
wherein the detectable marker is covalently bound to the oligonucleotide.

## Patentansprüche

1. Ein diagnostisches Kit zur Detektierung und/oder Diskriminierung zumindest eines Mikroorganismus des Genus *Enterococcus* in einer Probe durch Fluoreszenz-in-situ-Hybridisierung (fluorescence in situ hybridization (FISH)), umfassend
a) zumindest eine Oligonukleotid-Probe, die spezifisch für die Hybridisierung mit ribosomaler RNA von Mikroorganismen aus dem Genus *Enterococcus* ist, wobei besagte Probe zumindest eine Oligonukleotid-Sequenz der SEQ ID-Nr. 1 bis 16 umfasst,
SEQ ID-Nr. 1: GAA AGC GCC TTT CAC TCT TAT GC,
SEQ ID-Nr. 2: TAG GTG TTG TTA GCA TTT CG,
SEQ ID-Nr. 3: GAC TCC TTC AGA CTT ACT GCT TGG,
SEQ ID-Nr. 4: TTC ACA CAA TCG TAA CAT CCT A,
SEQ ID-Nr. 5: ATT CAC AAC TGT GTA ACA TCC TAT,
SEQ ID-Nr. 6: CAA AAG CGC CTT TCA AAC CGA,
SEQ ID-Nr. 7: TTG TCC GTA CAG GTA GAA GCA T,
SEQ ID-Nr. 8: AGC TTG TCC GTA CAG GTA GAG AA,
SEQ ID-Nr. 9: TCA CGC AAA CGT AAC ATC C,
SEQ ID-Nr. 10: TCA GCG GCA CCG TAA AG,
SEQ ID-Nr. 11: TTT TCC CGG TGG AGC AA,
SEQ ID-Nr. 12: CAG TTA CTC TCA CCC TTG TTC TT,
SEQ ID-Nr. 13: CAG TTC TCT GCG TCT ACC TC,
SEQ ID-Nr. 14: CAC CGC GGG TCC ATC CAT CA,
SEQ ID-Nr. 15: CCC TCT GAT GGG TAG GTT, and
SEQ ID-Nr. 16: CAA CTT TCT TCC ATG CGG AAA AT,
b) zumindest eine kompetitive Probe, umfassend ein Oligonukleotid mit einer Sequenz der SEQ ID-Nr. 17 bis 23,
SEQ ID-Nr. 17: CGA AAG CGC CTT TCA AAT CAA being competitive to SEQ ID NO. 6,
SEQ ID-Nr. 18: TTG TCC GTA CAG ATA GAA GCA T being competitive to SEQ ID NO. 7,
SEQ ID-Nr. 19: TCA CGC AGA CGT AAC ATC C being competitive to SEQ ID NO. 9,
SEQ ID-Nr. 20: TTT TTC CGG TGG AGC AA being competitive to SEQ ID NO. 11,
SEQ ID-Nr. 21: CGG CGC GGG TCC ATC CAG AA being competitive to SEQ ID NO. 14,
SEQ ID-Nr. 22: ACG CCG CGG GTC CAT CCA TWA being competitive to SEQ ID NO. 14, and
SEQ ID-Nr. 23: GTT TCA GTT CCC CRC GTC TAC CT being competitive to SEQ ID NO. 13,
c) zumindest eine weitere Oligonukleotid-Probe oder zumindest eine der besagten Oligonukleotid-Proben a) und b) mit zumindest einer Oligonukleotid-Sequenz der SEQ ID-Nr. 24
SEQ ID-Nr. 24: CCA GCT AGC GTG C,
wobei zumindest das Nukleotid in der unterstrichenen Position der SEQ ID-Nr. 24 eine blockierte Nukleinsäure (locked nucleic acid (LNA)) ist, so dass die Oligonukleotid-Probe spezifisch einen Mutationspunkt des Mikroorganismus unter Bedingungen detektiert, die zulassen, dass die Proben spezifisch hybridisieren, wobei die Bestimmung des Auftretens der in-situ-Hybridisierung zwischen besagten Proben und den Mikroorganismen in besagten Proben als Indikator der Linezolid-Widerstandsfähigkeit dient, und
d) zumindest eine weitere Oligonukleotid-Probe oder zumindest eine der besagten Oligonukleotid-Proben a) und b) mit zumindest einem Oligonukleotid umfassend eine Sequenz der SEQ ID-Nr. 25
SEQ ID-Nr. 25: CCA GCT CGC GTG C,
wobei zumindest das Nukleotid in der unterstrichenen Position der SEQ ID-Nr. 25 eine blockierte Nukleinsäure (locked nucleic acid (LNA)) ist, so dass die Oligonukleotid-Probe spezifisch einen Linezolid-Wildtyp detektiert.

2. Das diagnostische Kit gemäß Anspruch 1, wobei die Mikroorganismen ausgewählt sind aus den Genera *Enterococcus faecalis*, *Enterococcus faecium, Enterococcus gallinarium, Enterococcus avium, Enterococcus casseliflavus, Enterococcus flavescens, Enterococcus hirae*, *Enterococcus durans*, und *Enterococcus mundtii.*

3. Das diagnostische Kit gemäß einem der vorhergehenden Ansprüche, wobei besagte Probe a) zudem zumindest einen detektierbaren Marker umfasst.

4. Das diagnostische Kit gemäß Anspruch 3, wobei der detektierbare Marker kovalent an besagte Probe a) angebunden ist.

5. Das diagnostische Kit gemäß Anspruch 3 oder 4, wobei der detektierbare Marker ein Fluoreszenz-Marker, ein Chemolumineszenz-Marker, ein radioaktiver Marker, eine enzymatisch aktive Gruppe, ein Hapten und/oder eine Nukleinsäure, die durch Hybridisierung detektierbar ist, ist.

6. Das diagnostische Kit gemäß einem der vorhergehenden Ansprüche, wobei die kompetitive Probe nicht markiert ist.

7. Verfahren zur Detektierung und/oder Diskriminierung zumindest eines Mikroorganismus aus dem Genus *Enterococcus* in einer Probe, umfassend die Inkubierung der Probe mit den Oligonukleotid-Proben aus dem diagnostischen Kit gemäß einem der vorhergehenden Ansprüche und Bestimmung der Fluoreszenz-in-situ-Hybridisierung (fluorescence in situ hybridization (FISH)) in Gegenwart des Mikroorganismus, der mit der Probe hybridisiert ist, wobei das Oligonukleotid der besagten Probe a) die SEQ ID-Nr. 1 und/oder SEQ ID-Nr. 2 zur Detektierung des Mikroorganismus *Enterococcus faecalis* ist, und/oder
das Oligonukleotid der besagten Probe a) die SEQ ID-Nr. 3 und/oder SEQ ID-Nr. 4 zur Detektierung des Mikroorganismus *Enterococcus faecium* ist, und/oder
das Oligonukleotid der besagten Probe a) die SEQ ID-Nr. 5 zur Detektierung des Mikroorganismus *Enterococcus gallinarium* ist, und/oder das Oligonukleotid der besagten Probe a) die SEQ ID-Nr. 6 zur Detektierung des Mikroorganismus *Enterococcus avium* ist, und/oder
das Oligonukleotid der besagten Probe a) die SEQ ID-Nr. 7 und/oder SEQ ID-Nr. 8 zur Detektierung des Mikroorganismus *Enterococcus casseliflavus* ist, und/oder
das Oligonukleotid der besagten Probe a) die SEQ ID-Nr. 9 zur Detektierung des Mikroorganismus *Enterococcus hirae* und/oder *Enterococcus durans* ist, und/oder
das Oligonukleotid der besagten Probe a) die SEQ ID-Nr. 10 bis SEQ ID-Nr. 12 zur Detektierung des Mikroorganismus *Enterococcus mundtii* ist, und/oder
das Oligonukleotid der besagten Probe a) die SEQ ID-Nr. 13 zur Detektierung der Genera *Enterococcus spp*. und *Carnobacterium spp*. ist, und/oder das Oligonukleotid der besagten Probe a) die SEQ ID-Nr. 14 zur Detektierung der Genera *Enterococcus spp*., *Granulicatella spp*. und *Abiotrophia spp*. ist, und/oder
das Oligonukleotid der besagten Probe a) die SEQ ID-Nr. 15 zur Detektierung des Genus *Enterococcus spp*. ist, und/oder
das Oligonukleotid der besagten Probe a) die SEQ ID-Nr. 16 zur Detektierung der Genera der Van-C-Gruppe *enterococci* ist.

8. Das Verfahren gemäß Anspruch 7, umfassend weiter die Quantifizierung des zumindest einen Mikroorganismus.

9. Das Verfahren gemäß einem der Ansprüche 7 oder 8, wobei der zumindest eine Mikroorganismus der Genera der Van-C-Gruppe-Enterokokken zugehörig ist.

10. Das Verfahren gemäß einem der Ansprüche 7 bis 9, wobei der zumindest eine Mikroorganismus den Genera *Enterococcus faecalis*, *Enterococcus faecium, Enterococcus avium, Enterococcus hirae, Enterococcus durans,* und/oder *Enterococcus mundtii* zugehörig ist.

11. Das Verfahren gemäß einem der Ansprüche 7 bis 10, wobei die Probe weiter zumindest einen detektierbaren Marker, bevorzugt einen Fluoreszenz-Marker, einen Chemolumineszenz-Marker, einen radioaktiven Marker, eine enzymatisch aktive Gruppe, ein Hapten und/oder eine Nukleinsäure, die durch Hybridisierung detektierbar ist, umfasst.

12. Das Verfahren gemäß Anspruch 11, wobei der detektierbare Marker kovalent an das Oligonukleotid angebunden ist.

## Revendications

1. Kit de diagnostic pour détecter et/ou discriminer au moins un micro-organisme du genre *Enterococcus* dans un échantillon par hybridation *in situ* en fluorescence (FISH) comprenant
a) au moins une sonde oligonucléotidique spécifique pour l'hybridation avec l'ARN ribosomique des micro-organismes du genre *Enterococcus,* ladite sonde comprenant au moins un oligonucléotide ayant une séquence de SEQ ID NO. 1 à 16 :
SEQ ID NO. 1: GAA AGC GCC TTT CAC TCT TAT GC,
SEQ ID NO. 2: TAG GTG TTG TTA GCA TTT CG,
SEQ ID NO. 3: GAC TCC TTC AGA CTT ACT GCT TGG,
SEQ ID NO. 4: TTC ACA CAA TCG TAA CAT CCT A,
SEQ ID NO. 5: ATT CAC AAC TGT GTA ACA TCC TAT,
SEQ ID NO. 6: CAA AAG CGC CTT TCA AAC CGA,
SEQ ID NO. 7: TTG TCC GTA CAG GTA GAA GCA T,
SEQ ID NO. 8: AGC TTG TCC GTA CAG GTA GAG AA,
SEQ ID NO. 9: TCA CGC AAA CGT AAC ATC C,
SEQ ID NO. 10: TCA GCG GCA CCG TAA AG,
SEQ ID NO. 11: TTT TCC CGG TGG AGC AA,
SEQ ID NO. 12: CAG TTA CTC TCA CCC TTG TTC TT,
SEQ ID NO. 13: CAG TTC TCT GCG TCT ACC TC,
SEQ ID NO. 14: CAC CGC GGG TCC ATC CAT CA,
SEQ ID NO. 15: CCC TCT GAT GGG TAG GTT, et
SEQ ID NO. 16: CAA CTT TCT TCC ATG CGG AAA AT,
b) au moins une sonde concurrente comprenant un oligonucléotide ayant une séquence de SEQ ID NO. 17 à 23 :
SEQ ID NO. 17: CGA AAG CGC CTT TCA AAT CAA qui est concurrente de la SEQ ID NO. 6,
SEQ ID NO. 18: TTG TCC GTA CAG ATA GAA GCA T qui est concurrente de la SEQ ID NO. 7,
SEQ ID NO. 19: TCA CGC AGA CGT AAC ATC C qui est concurrente de la SEQ ID NO. 9,
SEQ ID NO. 20: TTT TTC CGG TGG AGC AA qui est concurrente de la SEQ ID NO. 11,
SEQ ID NO. 21: CGG CGC GGG TCC ATC CAG AA qui est concurrente de la SEQ ID NO. 14,
SEQ ID NO. 22: ACG CCG CGG GTC CAT CCA TWA qui est concurrente de la SEQ ID NO. 14, et
SEQ ID NO. 23: GTT TCA GTT CCC CRC GTC TAC CT qui est concurrente de la SEQ ID NO. 13,
c) au moins une autre sonde oligonucléotidique ou au moins l'une desdites sondes oligonucléotidique a) et b) avec au moins un oligonucléotides comprenant une séquence de SEQ ID NO. 24 :
SEQ ID NO. 24: CCA GCT AGC GTG C,
et au moins le nucléotide à la position soulignée dans la SEQ ID NO 24 est un acide nucléique bloqué (LNA) de sorte que la sonde oligonucléotidique détecte de manière spécifique une mutation ponctuelle du micro-organisme dans des conditions qui permettent à la sonde de s'hybrider de manière spécifique, et déterminer la présence d'une hybridation *in situ* entre lesdites sondes et le micro-organisme dans ledit échantillon en tant qu'une indication d'une résistance au linézolide, et
d) au moins une autre sonde oligonucléotidique ou au moins l'une desdites sondes oligonucléotidiques a) et b) avec au moins un oligonucléotide comprenant une séquence de SEQ ID NO 25. :
SEQ ID NO. 25: CCA GCT CGC GTG C,
et au moins le nucléotide à la position soulignée dans la SEQ ID NO. 25 est un acide nucléique bloqué (LNA) de sorte que la sonde oligonucléotidique détecte de manière spécifique un type sauvage de linézolide.

2. Kit de diagnostic selon la revendication 1, où les micro-organismes sont choisis parmi les genres
*Enterococcus faecalis*, *Enterococcus faecium, Enterococcus gallinarium, Enierococcus avium, Enterococcus casseliflavus, Enterococcus flavescens, Enterococcus hirae*, *Enterococcus durans*, et *Enterococcus mundtii*.

3. Kit de diagnostic selon l'une quelconque des revendications précédentes, où ladite sonde a) comprend en outre au moins un marqueur détectable.

4. Kit de diagnostic selon la revendication 3, où le marqueur détectable est lié de manière covalente à ladite sonde a).

5. Kit de diagnostic selon la revendication 3 ou 4, où le marqueur détectable est un marqueur fluorescent, un marqueur chimioluminescent, un marqueur radioactif, un groupe actif de manière enzymatique, un haptène et/ou un acide nucléique détectable par hybridation.

6. Kit de diagnostic selon l'une quelconque des revendications précédentes, où la sonde concurrente n'est pas marquée.

7. Procédé pour détecter et/ou discriminer au moins un micro-organisme du genre *Enterococcus* dans un échantillon, consistant à incuber l'échantillon avec les sondes oligonucléotidiques du kit de diagnostic selon l'une des revendications précédentes et à déterminer par hybridation *in situ* en fluorescence (FISH) la présence de micro-organismes hybridés avec la sonde, l'oligonucléotide de ladite sonde a) étant la SEQ ID NO. 1 et/ou la SEQ ID NO. 2 détectant le micro-organisme *Enterococcus faecalis* et/ou
l'oligonucléotide de ladite sonde a) étant la SEQ ID NO. 3 et/ou la SEQ ID NO. 4 détectant le micro-organisme *Enterococcus faecium* et/ou
l'oligonucléotide de ladite sonde a) étant la SEQ ID NO. 5 détectant le micro-organisme *Enterococcus gallinarium* et/ou
l'oligonucléotide de ladite sonde a) étant la SEQ ID NO. 6 détectant le micro-organisme *Enterococcus avium* et/ou
l'oligonucléotide de ladite sonde a) étant la SEQ ID NO. 7 et/ou la SEQ ID NO. 8 détectant le micro-organisme *Enterococcus casseliflavus* et/ou
l'oligonucléotide de ladite sonde a) étant la SEQ ID NO. 9 détectant le micro-organisme *Enterococcus hirae* et/ou *Enterococcus durans* et/ou
l'oligonucléotide de ladite sonde a) étant les SEQ ID NO. 10 à SEQ ID NO. 12 détectant le micro-organisme *Enterococcus mundtii* et/ou
l'oligonucléotide de ladite sonde a) étant là SEQ ID NO. 13 détectant les genres *Enterococcus spp*. et *Carnobacterium spp*. et/ou
l'oligonucléotide de ladite sonde a) étant la SEQ ID NO. 14 détectant les genres *Enterococcus spp*., *Granulicatella spp*. et *Abiotrophia spp*. et/ou
l'oligonucléotide de ladite sonde a) étant la SEQ ID NO. 15 détectant le genre *Enterococcus spp*. et/ou
l'oligonucléotide de ladite sonde a) étant la SEQ ID NO. 16 détectant les genres *enterococci* du groupe Van-C.

8. Procédé selon la revendication 7, consistant en outre à quantifier l'au moins un micro-organisme.

9. Procédé selon la revendication 7 ou 8, où au moins un micro-organisme appartient aux genres *enterococci* du groupe Van-C.

10. Procédé selon l'une des revendications 7 à 9, où l'au moins un micro-organisme appartient aux genres *Enterococcus faecalis*, *Enterococcus faecium, Enterococcus avium*, *Enterococcus hirae*, *Enterococcus durans*, et/ou *Enterococcus mundtii.*

11. Procédé selon l'une quelconque des revendications 7 à 10, où la sonde comprend en outre au moins un marqueur détectable, de préférence un marqueur fluorescent, un marqueur chimioluminescent, un marqueur radioactif, un groupe actif de manière enzymatique, un haptène et/ou un acide nucléique détectable par hybridation.

12. Procédé selon la revendication 11, où le marqueur détectable est lié de manière covalente à l'oligonucléotide.


### SEQUENCE LISTING

<110> University of ulm
<120> An oligonucleotide probe and a process of detecting or discriminating microorganisms in a sample
<130> 079EP 0859
<160> 25
<170> PatentIn version 3.3
<210> 1
   <211> 23
   <212> DNA
   <213> Artificial
<220>
   <223> oligonucleotide
<400> 1
   gaaagcgcct ttcactctta tgc 23
<210> 2
   <211> 20
   <212> DNA
   <213> Artificial
<220>
   <223> oligonucleotide
<400> 2
   taggtgttgt tagcatttcg 20
<210> 3
   <211> 24
   <212> DNA
   <213> Artificial
<220>
   <223> oligonucleotide
<400> 3
   gactccttca gacttactgc ttgg 24
<210> 4
   <211> 22
   <212> DNA
   <213> Artificial
<220>
   <223> oligonucleotide
<400> 4
   ttcacacaat cgtaacatcc ta 22
<210> 5
   <211> 24
   <212> DNA
   <213> Artifiicial
<400> 5
   attcacaact gtgtaacatc ctat 24
<210> 6
   <211> 21
   <212> DNA
   <213> Artificial
<220>
   <223> oligonucleotide
<400> 6
   caaaagcgcc tttcaaaccg a 21
<210> 7
   <211> 22
   <212> DNA
   <213> Artificial
<220>
   <223> oligonucleotide
<400> 7
   ttgtccgtac aggtagaagc at 22
<210> 8
   <211> 23
   <212> DNA
   <213> Artificial
<220>
   <223> oligonucleotide
<400> 8
   agcttgtccg tacaggtaga gaa 23
<210> 9
   <211> 19
   <212> DNA
   <213> Artificial
<220>
   <223> oligonucleotide
<400> 9
   tcacgcaaac gtaacatcc 19
<210> 10
   <211> 17
   <212> DNA
   <213> Artificial
<220>
   <223> oligonucleotide
<400> 10
   tcagcggcac cgtaaag 17
<210> 11
   <211> 17
   <212> DNA
   <213> Artificial
<220>
   <223> oligonucleotide
<400> 11
   ttttcccggt ggagcaa 17
<210> 12
   <211> 23
   <212> DNA
   <213> Artificial
<220>
   <223> oligonucleotide
<400> 12
   cagttactct cacccttgtt ctt 23
<210> 13
   <211> 20
   <212> DNA
   <213> Artificial
<220>
   <223> oligonucleotide
<400> 13
   cagttctctg cgtctacctc 20
<210> 14
   <211> 20
   <212> DNA
   <213> Artificial
<220>
   <223> oligonucleotide
<400> 14
   caccgcgggt ccatccatca 20
<210> 15
   <211> 18
   <212> DNA
   <213> Artificial
<220>
   <223> oligonucleotide
<400> 15
   ccctctgatg ggtaggtt 18
<210> 16
   <211> 23
   <212> DNA
   <213> Artificial
<220>
   <223> oligonucleotide
<400> 16
   caactttctt ccatgcggaa aat 23
<210> 17
   <211> 21
   <212> DNA
   <213> Artificial
<220>
   <223> oligonucleotide
<400> 17
   cgaaagcgcc tttcaaatca a 21
<210> 18
   <211> 22
   <212> DNA
   <213> Artificial
<220>
   <223> oligonucleotide
<400> 18
   ttgtccgtac agatagaagc at 22
<210> 19
   <211> 19
   <212> DNA
   <213> Artificial
<220>
   <223> oligonucleotide
<400> 19
   tcacgcagac gtaacatcc 19
<210> 20
   <211> 17
   <212> DNA
   <213> Artificial
<220>
   <223> oligonucleotide
<400> 20
   tttttccggt ggagcaa 17
<210> 21
   <211> 20
   <212> DNA
   <213> Artificial
<220>
   <223> oligonucleotide
<400> 21
   cggcgcgggt ccatccagaa 20
<210> 22
   <211> 21
   <212> DNA
   <213> Artificial
<220>
   <223> oligonucleotide
<400> 22
   acgccgcggg tccatccatw a 21
<210> 23
   <211> 23
   <212> DNA
   <213> Artificial
<220>
   <223> oligonucleotide
<400> 23
   gtttcagttc cccrcgtcta cct 23
<210> 24
   <211> 13
   <212> DNA
   <213> Artificial
<220>
   <223> oligonucleotide
<400> 24
   ccagctagcg tgc 13
<210> 25
   <211> 13
   <212> DNA
   <213> Artificial
<220>
   <223> oligonucleotide
<400> 25
   ccagctcgcg tgc 13